# EUROPÄISCHE PATENTSCHRIFT

(11) **EP 3 876 877 B1**
(45) Veröffentlichungstag und Bekanntmachung des Hinweises auf die Patenterteilung: **23.09.2026**
(21) Anmeldenummer: 19798063.4
(22) Anmeldetag: 04.11.2019
(51) Int. Cl.: A61F 5/01

(54) **VORRICHTUNG ZUM UNTERSTÜTZEN WENIGSTENS EINES ARMES EINES BENUTZERS**
DEVICE FOR SUPPORTING AT LEAST ONE ARM OF A USER
DISPOSITIF POUR LE SOUTIEN D'AU MOINS UN BRAS D'UN UTILISATEUR

(30) Priorität: 05.11.2018 DE 102018127553
(43) Veröffentlichungstag der Anmeldung: 15.09.2021
(73) Patentinhaber: Ottobock SE & Co. KGaA, 37115 Duderstadt (DE)
(72) Erfinder: KURZWEG, Annedore, 37085 Göttingen (DE); MIZERA, Oliver, 34134 Kassel (DE); KRUCHEM, Tim, 37073 Göttingen (DE); VOLLBRECHT, Matthias, 37412 Herzberg (DE); RÖSCHEL, Fabienne, 37073 Göttingen (DE); KROLL-ORYWAHL, Olaf, 37154 Northeim (DE); MLADEK, Markus, 37130 Gleichen (DE); WIEDERHOLD, René, 37339 Breitenworbis (DE); TÜTTEMANN, Markus, 45731 Waltrop (DE); BREMER, Michael, 37083 Göttingen (DE); MERKER, Maximilian, 42349 Wuppertal (DE); FINKE, Lars Benjamin, 37136 Landolfshausen (DE); LIDOLT, Klaus, 37115 Duderstadt (DE); WEHSELY-SWICZINSKY, Adam, 1020 Wien (AT); RITSCHL, Matthias, 1030 Wien (AT)
(74) Vertreter: Gramm, Lins & Partner Patent- und Rechtsanwälte PartGmbB
(86) Internationale Anmeldenummer: PCT/EP2019/080059
(87) Internationale Veröffentlichungsnummer: WO 2020/094549

(56) Entgegenhaltungen:
- DE-A1- 102016 121 203
- US-A1- 2016 081 871
- US-A1- 2016 339 583

## Beschreibung

Die Erfindung betrifft eine Vorrichtung zum Unterstützen wenigstens eines Armes eines Benutzers, wobei die Vorrichtung wenigstens ein Armstützelement mit je einer Armschale zum Anlegen an jeweils einen Arm, wenigstens einen passiven Aktuator, der als elastisches Element ausgebildet und eingerichtet ist, eine Kraft auf wenigstens eines der Armstützelemente auszuüben, durch die im angelegten Zustand der Vorrichtung eine Aufwärtsbewegung des Arms in der Armschale unterstützt wird, und wenigstens ein Gegenlager für die aufzubringende Kraft aufweist.

Eine derartige Vorrichtung ist beispielsweise aus der US 2016/0081871 A1 bekannt. Sie verfügt über ein Gegenlagerelement, das in Form eines um den Rumpf des Benutzers herum legbaren Gurtes ausgebildet ist. An ihm befinden sich zwei entlang des Rückens zur Schulter verlaufende Stützstreben, die oberhalb und seitlich neben den Schultern des Benutzers mit jeweils einem Gelenk verbunden sind, so dass der Arm angehoben werden kann. An den entsprechenden Gelenken sind Federelemente angeordnet, durch die eine nach oben gerichtete Kraft auf die Armschalen ausgeübt werden kann, so dass beispielsweise beim Heben schwerer Gegenstände oder beim Arbeiten über Kopf eine Unterstützung der Arme erfolgt. Sollen die Arme gesenkt werden, muss durch die Arme ein Druck auf die Armschalen ausgeübt werden, der die von den Federelementen aufgebrachte Kraft übersteigt, so dass die Arme absinken.

Aus der DE 10 2016 121203 A1 ist eine Vorrichtung bekannt, bei der der Kraftverlauf, also die von dem Aktuator als Funktion des Winkels des Arms aufgebrachte Kraft, über Konturscheiben, über die ein Bowdenzug verläuft, einstellbar ist.

Aus der WO 2014/0093408 A2 und der US 9,427,865 B2 sind ähnliche Vorrichtungen bekannt, bei denen als mechanische Energiespeicher, der als passive Aktuator wirkt, jeweils eine Feder, insbesondere eine Zugfeder vorgesehen ist, die mit einem Bowdenzug verbunden ist. Dieser wird über eine Umlenkrolle geführt, so dass beim Verschwenken des Armes, was eine Bewegung des Armstützelementes relativ zum Gegenlagerelement bedeutet, die Feder gedehnt wird, so dass der mechanische Energiespeicher mit Energie aufgeladen wird.

Eine aktive Vorrichtung, die Arme beim Arbeiten über Kopf unterstützt, ist aus der EP 3 156 193 A1 bekannt. Die Armschalen sind über eine Vielzahl verschiedener Gelenke und Rahmenelemente miteinander verbunden. Dadurch sollen möglichst viele Bewegungen, die ein Schultergelenk durchführen kann, auch mit der angelegten Vorrichtung möglich sein. Weitere Unterstützungsvorrichtungen, die insbesondere beim Heben von schweren Gegenständen oder beim Arbeiten über Kopf unterstützen, sind beispielsweise aus der WO 2014/195373 A1 und der US 2016/339583 A1 bekannt.

Nachteilig bei all diesen Vorrichtungen ist, dass die auf die jeweilige Armschale ausgeübte Kraft permanent vorhanden ist. Dies bedeutet insbesondere, dass sich beim Anlegen der Vorrichtung die Armstützelemente in der nach oben gestreckten Position befinden, so dass sie zum Einlegen des Armes in die jeweilige Armschale erst nach unten gedrückt werden müssen. Dies ist umständlich und benötigt Platz, so dass die Gefahr besteht, mit den ausgestreckten Armstützelementen gegen andere Gegenstände zu schlagen.

Zudem kommt es beim Ablegen der Vorrichtung in dem Moment, wo der Arm aus der Armschale entfernt wird, zu einer schnellen Bewegung der Armstützelemente nach oben, wodurch Beschädigungen anderer Gegenstände oder sogar Verletzungen des Benutzers hervorgerufen werden können.

Aus der US 2017/0173783 A1 ist ein Wirkprinzip bekannt, bei dem dieses Problem nicht auftritt. Anders als bei den hier beschriebenen Vorrichtungen ist der Aktuator nicht in der Lage, eine Kraft auf das Armstützelement aufzubringen, durch die eine Aufwärtsbewegung des Arms in der Armschale unterstützt wird. Vielmehr wird eine mechanische Feder ausschließlich dazu verwendet, eine Abwärtsbewegung zu erschweren. Wird ein in dieser Vorrichtung angeordneter Arm nicht abgesenkt, wirkt daher auch keine Kraft. Insbesondere beim Anheben von schweren Gegenständen ist diese Vorrichtung anders als die anderen vorher genannten Ausführungsformen daher keine Hilfe.

Der Erfindung liegt daher die Aufgabe zugrunde, diese Nachteile zu beheben oder zumindest zu mindern.

Die Erfindung löst die gestellte Aufgabe durch eine Vorrichtung gemäß dem Oberbegriff des Anspruchs 1, die sich dadurch auszeichnet, dass sie wenigstens ein Betätigungselement aufweist, durch dessen Betätigen der Aktuator in einen ersten Zustand, in dem der Aktuator die Kraft auf das Armstützelement ausübt, und in einen zweiten Zustand bringbar ist, in dem er eine kleinere oder keine Kraft auf das wenigstens eine Armstützelement ausübt, wobei durch das Betätigen des Betätigungselementes Einrichtung der vom Aktuator aufgebrachten Kraft veränderbar ist, ohne dass ein Ende des elastischen Elementes verschoben wird. Bevorzugt verfügt die Vorrichtung über wenigstens ein Kraftübertragungselement, das eingerichtet ist, eine Gegenkraft von dem wenigstens einen Armstützelement auf das Gegenlagerelement zu übertragen.

Im nicht angelegten Zustand der Vorrichtung befindet sich der Aktuator vorteilhafterweise im zweiten Zustand, so dass besonders bevorzugt keine Kraft auf das Armstützelement ausgeübt wird. Der Benutzer der Vorrichtung kann diese folglich anlegen und die Armstützelemente mit den Armschalen in die zum Anlegen bequemste Position bringen. Dabei sind die Armstützelemente in der Regel nach unten ausgerichtet, so dass der jeweilige Arm in die Armschale leicht eingelegt wer-den kann. Sobald die Arme in den Armschalen anliegen und die Armschalen gegebenenfalls um den Arm herum geschlossen wurden, befindet sich die Vorrichtung im angelegten Zustand. In diesem Fall kann durch Betätigen des wenigstens einen Betätigungselementes der Aktuator in den ersten Zustand gebracht werden, indem er die Unterstützungskraft auf wenigstens der Armstützelemente aufbringt.

Vorzugsweise übt der Aktuator im ersten Zustand eine maximale Kraft auf das wenigstens eine Armstützelement aus. Der erste Zustand ist folglich bevorzugt so definiert, dass eine weitere Krafterhöhung durch den Aktuator insbesondere durch Betätigen des wenigstens einen Betätigungselementes nicht möglich ist.

Vorzugsweise ist der Aktuator durch Betätigen des wenigstens einen Betätigungselementes in mehreren Stufen oder stufenlos von dem ersten Zustand in den zweiten Zustand bringbar, wobei die auf das wenigstens eine Armstützelement durch den Aktuator ausgeübte Kraft abnimmt. Besonders bevorzugt ist der Aktuator durch Betätigen des wenigstens einen Betätigungselementes in wenigstens einen Zwischenzustand, bevorzugt jedoch mehrere Zwischenzustände, bringbar, so dass die auf das wenigstens eine Armstützelement durch den Aktuator ausgeübte Kraft in Stufen oder stufenlos einstellbar ist. Das Betätigungselement ist vorzugsweise an einer Unterseite der Armschale, an einem Gelenk, durch das das Armstützelement und das Kraftübertragungselement schwenkbar aneinander angeordnet sind, oder seitlich außen an der Armschale oder dem Armstützelement angeordnet. Mit der Unterseite der Armschale wird dabei die Seite bezeichnet, die im angelegten Zustand von dem Arm des Benutzers abgewandt ist. Sie ist bei nicht angehobenem Arm zwischen dem Rumpf und dem Arm angeordnet. Seitlich außen bezeichnet die Seite, die im angelegten Zustand bei nicht angehobenem Arm dem Rumpf abgewandt ist.

Auf diese Weise lässt sich die vom Aktuator ausgeübte Kraft frei wählen und an die persönlichen Bedürfnisse des Trägers der Vorrichtung anpassen. Wird beispiels-weise ein Kraftangriffspunkt, an dem der Aktuator an das Armstützelement angreift, relativ zu einer Schwenkachse, um die das Armstützelement beispiels-weise relativ zum Kraftübertragungselement verschwenkbar ist, verschoben, ändert sich auch der entsprechende Hebel, so dass das aufgebrachte Drehmoment verändert wird. Dies wird unter einer Veränderung der "ausgeübten Kraft" mit verstanden.

Vorteilhafterweise weist der wenigstens eine Aktuator wenigstens ein elastisches Element auf, das entspannbar ist, indem der Aktuator durch Betätigen des wenigstens einen Betätigungselementes aus dem ersten Zustand in den zweiten Zustand gebracht wird. Das elastische Element kann beispielsweise eine Feder, beispiels-weise eine Zugfeder, ein elastisches Element, beispielsweise ein Expander, oder ein sonstiges Element sein, das vorzugsweise in ersten Zustand des Aktuators vor-gespannt ist und für die ausgeübte Kraft verantwortlich ist.

Vorteilhafterweise befindet sich an einem Ende des elastischen Elementes ein Befestigungselement, das durch Betätigen des wenigstens einen Betätigungselementes bewegbar, insbesondere verschwenkbar oder verschiebbar ist. Vorzugsweise erfolgt diese Bewegung des Endes des elastischen Elementes gedämpft, beispiels-weise durch ein Federelement, ein Dämpfungselement und/oder eine kontrolliert aufgebrachte Reibkraft. Dadurch wird ein unkontrolliertes Zurückschnellen des elastischen Elementes, das beispielsweise ein Expander sein kann, verhindert.

Vorzugsweise wird das Ende des elastischen Elementes in der ersten Position, in der der Aktuator die maximale Kraft auch das Armstützelement ausübt arretiert. Dies geschieht vorzugsweise automatisch wenn und indem die erste Position erreicht wird. Dazu kann beispielsweise ein modifizierter "push-push"-Mechanismus und/oder ein Umschalthebel verwendet werden. Durch nochmaliges Betätigen des Betätigungselementes ("Überziehen") wird die Verriegelung wieder gelöst und der Aktuator kann in den zweiten Zustand gebracht werden.

Vorzugsweise ist eine Sicherungseinrichtung beispielsweise in Form einer Rasteinrichtung mit einem Rastelement und einer Rasthinterschneidung vorhanden, durch die sichergestellt wird, dass der Aktuator nur dann von dem ersten Zustand in den zweiten Zustand und/oder umgekehrt bringbar ist, wenn die Vorrichtung angelegt ist.

Das Betätigungselement ist vorzugsweise selbsthemmend.

Das Betätigungselement kann beispielsweise ein Seilzug, eine Schnur oder ein Gurt sein, das an dem Befestigungselement angeordnet ist. Das Befestigungselement selbst ist beispielsweise entlang einer Stange, die auch als Kraftübertragungselement für die vom Aktuator aufgebrachte Kraft dienen kann, verschiebbar gelagert. Ein Ende des Betätigungselementes ist an einem anderen Bauteil der Vorrichtung, beispielsweise einem um den Rumpf herum gelegten Gurt, befestigbar. Dafür sind beispielsweise Formschlusselemente in Form von Druckknöpfen oder Klettverschlusselementen vorhanden. Beim Anlegen der Vorrichtung ist dieses Ende des Betätigungselementes beispielsweise nicht an einem anderen Bauteil der Vorrichtung festgelegt oder in einer solchen Position befestigt, dass das elastische Element entspannt ist. Nach dem Anlegen der Vorrichtung, wenn die Armschalen um den Arm geschlossen oder der Arm zumindest in den Armschalen angeordnet ist, kann auf das Betätigungselement ein Zug ausgeübt werden, wodurch das Befestigungselement, an dem sich das Ende des elastischen Elementes befindet, verschoben wird. Dadurch wird auch das Ende des elastischen Elementes verschoben und das elastische Element auf diese Weise gedehnt und somit gespannt. In dieser Position wird das Ende des Betätigungselementes über die bereits genannten Mechanismen festgelegt, so dass das das Befestigungselement und damit das Ende des elastischen Elementes ebenfalls fixiert ist. In diesem Fall befindet sich der Aktuator im ersten Zustand und bringt eine Kraft auf das Armstützelement auf.

Soll die Vorrichtung abgelegt werden, kann das Ende des Betätigungselementes gelöst werden. Durch die Kraft des elastischen Elementes wird das Ende des elastischen Elementes, an dem sich das Befestigungselement und das Betätigungselement befindet, verschoben, so dass das elastische Element entspannt wird und sich der Aktuator im zweiten Zustand befindet. Danach kann die Vorrichtung gefahrlos abgelegt werden, ohne dass es zu ruckartigen Bewegungen kommt.

Besonders vorteilhafterweise kann das Betätigungselement vom Benutzer der Vor-richtung betätigt werden, wenn der Benutzer die Vorrichtung angelegt hat.

Alternativ zum Zugelement, beispielsweise Gurt oder Schnur, ausgebildeten Betätigungselementes kann auch ein Umlenkhebel, ein Spannhebel oder eine sonstige Vorrichtung zum Verschieben des Endes des elastischen Elementes vorhanden sein. Diese Vorrichtung ist vorzugsweise selbsthemmend.

Alternativ oder zusätzlich kann das Betätigungselement als Drehknopf ausgebildet sein. Eine derartige Ausgestaltung des Betätigungselementes wird beispielsweise von der Firma BOA angeboten.

Alternativ oder zusätzlich dazu verfügt das Gegenlager über wenigstens ein Gegenlagerelement und wenigstens zwei Kraftübertragungselemente, wobei jedes Armstützelement um eine Schwenkachse schwenkbar an einem der Kraftübertragungselemente angeordnet ist. Das Gegenlagerelement kann beispielsweise ein Hüftgurt, der selbstverständlich gepolstert sein kann, oder ein sonstiges am Körper des Benutzers angeordnetes Element sein. An diesem befinden sich die beiden Kraftübertragungselemente, die beispielsweise eine nach oben gerichtete Stange sein können. An dem dem Gegenlagerelement entgegengesetzten Ende der Stange befindet sich ein Schwenkgelenk mit einer Schwenkachse, über das das jeweilige Armstützelement am Kraftübertragungselement angeordnet ist.

Vorzugsweise verläuft die von dem Aktuator ausgeübte Kraft entlang einer Kraftrichtung, die im zweiten Zustand des Aktuators näher an der Schwenkachse verläuft als im ersten Zustand des Aktuators. Die Kraft selbst greift beispielsweise an einem Hebel an, der mit dem Armstützelement fest verbunden oder Teil des Armstützelementes ist, um von der Schwenkachse des Schwenkgelenkes beabstandet ist. Auf diese Weise wird durch die vom Aktuator aufgebrachte Kraft ein Drehmoment auf das Armstützelement um die Schwenkachse herum aufgebracht, das zu der unterstützenden Kraft führt. Je länger das Hebelelement ist, an dem die Kraft angreift, desto größer ist das Drehmoment und damit die resultierende Kraft. Durch das Betätigungselement ist es vorteilhafterweise möglich, diese Kraftrichtung zu verändern und im zweiten Zustand des Aktuators näher an die Schwenk-achse heran zu verlagern. Besonders vorteilhafterweise verläuft die Kraftrichtung im zweiten Zustand durch die Schwenkachse. Ist der wenigstens eine passive Aktuator beispielsweise ein elastisches Element, beispielsweise eine Feder oder Zugfeder, ist es in diesem Fall nicht nötig, ein Ende des Elementes zu verschieben und den Aktuator zu entspannen. Vielmehr wird die Richtung des Aktuators oder lediglich die Richtung der aufgebrachten Kraft so verändert, dass sie vom Kraftangriffspunkt, der beispielsweise an dem bereits benannten Hebel angeordnet ist, in Richtung auf die Schwenkachse verläuft. Eine solche Kraft hat kein Drehmoment um die Schwenkachse zur Folge, so dass auch keine unterstützende Kraft auf das Armstützelement ausgeübt wird.

Vorteilhafterweise verfügt der Aktuator über ein elastisches Element, an dem wenigstens ein Zugelement angeordnet ist, auf das durch Betätigen des Betätigungselementes eine Zugkraft ausübbar ist, wenn sich der Aktuator im ersten Zustand befindet. Ein derartiges Zugelement ist beispielsweise ein Seil, ein Draht oder ein Gurt. Das Betätigungselement kann beispielsweise ein Wickelmechanismus sein, über den das Zugelement aufwickelbar ist. Der Abstand zwischen den beispiels-weise an dem Armstützelement, insbesondere der Armschiene, angeordneten Betätigungselement und der Verbindung zwischen dem Zugelement und dem elastischen Elementen kann auf diese Weise verkleinert werden. Dadurch kann durch geschickte Anordnung der einzelnen Bauteile die Verbindung zwischen dem Zugelement und dem elastischen Element näher an die Schwenkachse des Schwenkgelenkes heranbewegt werden und im Optimalfall mit dieser in Überdeckung gebracht werden.

Soll der Aktuator in diesem Ausführungsbeispiel zurück in die erste Position gebracht werden, ist es beispielsweise ausreichend, wenn der Wickelmechanismus gelöst werden kann und durch das immer noch gespannte elastische Element eine Zugkraft auf das Zugelement in die entgegengesetzte Richtung aufgebracht wird. Dadurch wird der Aktuator wieder in den ersten Zustand zurückbewegt.

Vorteilhafterweise verfügen die Armschalen über jeweils ein Verschlusselement, durch das die Armschale um den Arm des Benutzers schließbar ist. Auf diese Weise wird verhindert, dass der Arm versehentlich aus der jeweiligen Armschale herausrutscht oder herausbewegt wird, was zu einer unkontrollierten Bewegung des Armstützelementes führen würde.

Besonders vorteilhafterweise kann das Verschlusselement nur dann geöffnet wer-den, wenn sich der Aktuator im zweiten Zustand befindet. Dafür kann beispiels-weise eine mechanische Verriegelung oder Entriegelung vorhanden sein. Auf diese Weise wird sicher verhindert, dass die Vorrichtung abgelegt werden kann, wenn sich der Aktuator im ersten Zustand befindet.

Besonders bevorzugt verfügt die Vorrichtung über zwei Armstützelemente mit jeweils einer Armschale zum Anlegen an jeweils einen Arm und besonders bevorzugt über zwei Kraftübertragungselemente. Als besonders vorteilhaft hat sich herausgestellt, wenn die Vorrichtung zudem über wenigstens zwei Aktuatoren verfügt, um die auf die verschiedenen Armstützelemente aufzubringende Unterstützungskräfte individuell einstellen zu können. Die Aktuatoren sind vorzugsweise über jeweils wenigstens ein Betätigungselement, besonders bevorzugt unabhängig voneinander in einen ersten Zustand und einen zweiten Zustand bringbar, wobei im zweiten Zustand der Aktuator eine kleinere oder keine Kraft auf das Armstützelement ausübt als im ersten Zustand.

Mit Hilfe der beiliegenden Figuren wird nachfolgend ein Ausführungsbeispiel der vorliegenden Erfindung näher erläutert. Es zeigt:
- Figur 1 -: die schematische Darstellung eines Teils einer Vorrichtung gemäß einem ersten Ausführungsbeispiel der vorliegenden Erfindung,
- Figur 2 -: die schematische Darstellung einer alternativen Ausführungsform,
- Figur 3 bis 5 -: weitere Ausführungsformen,
- Figur 6 -: die schematische Darstellung einer weiteren Ausführungsform der vorliegenden Erfindung,
- Figur 7 -: die Vorrichtung aus Figur 6 mit dem Aktuator im zweiten Zustand,
- Figur 8 -: die schematische Darstellung eines weiteren Ausführungsbeispiels,
- Figur 9 -: die Darstellung aus Figur 8 mit dem Aktuator im zweiten Zustand,
- Figur 10 -: die schematische Darstellung einer Sicherungseinrichtung und
- Figur 11 bis 16 -: schematische Darstellungen verschiedener Ausführungsformen.

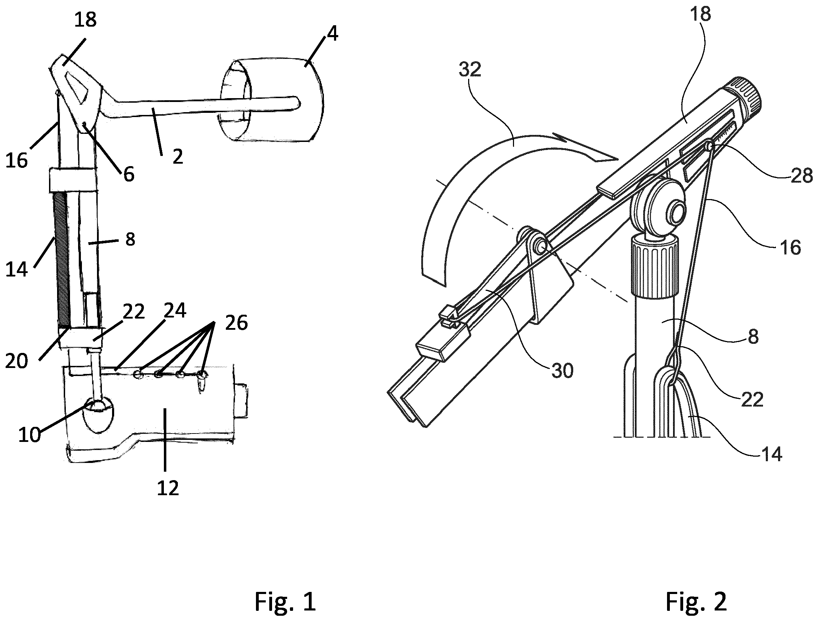

Figur 1 zeigt schematisch einen Teil einer Vorrichtung gemäß einem ersten Ausführungsbeispiel der vorliegenden Erfindung. Gezeigt ist ein Armstützelement 2, an dem sich eine Armschale 4 befindet. Über ein Schwenkgelenk 6 ist das Armstützelement 2 schwenkbar an einem Kraftübertragungselement 8 angeordnet, das mit seinem unterem Ende 10 an einem Gegenlagerelement 12 angeordnet ist. Bei dem Gegenlagerelement 12 handelt es sich um einen Bauchgurt oder Hüftgurt, der um den Körper des Trägers der Vorrichtung herumgelegt wird.

Am Kraftübertragungselement befindet sich ein passiver Aktuator 14, der im gezeigten Ausführungsbeispiel als elastisches Element dargestellt ist. Es ist über einen Bowdenzug 16 mit einem Hebelelement 18 verbunden, an dem sich das Armstütz-element 2 befindet. Ein Ende 20 des passiven Aktuators 14 ist mit einem Befestigungselement 22 verschiebbar am Kraftübertragungselement 8 angeordnet. Am Befestigungselement 22 befindet sich ein Betätigungselement 24 in Form eines Bandes oder Gurtes, über das eine Zugkraft auf das Befestigungselement 22 und damit das Ende 20 des passiven Aktuators 14 ausgeübt werden kann. In verschiedenen Befestigungspositionen 26 sind am Gegenlagerelement 12 Formschlusselement, beispielsweise Klettverschlusselemente, Druckknöpfe oder sonstige Befestigungselemente angeordnet, so dass das Betätigungselement 24 in unter-schiedlichen Positionen am Gegenlagerelement 12 festlegbar ist. Dadurch ist einerseits der passive Aktuator vom in Figur 1 gezeigten ersten Zustand in den zweiten Zustand und umgekehrt bringbar und andererseits eine Vorspannung, die durch den passiven Aktuator ausgeübt wird, einstellbar.

Figur 2 zeigt eine alternative Ausführungsform. Der Bowdenzug 16, der am passiven Aktuator 14 angeordnet ist, wird über eine Umlenkrolle 28 geführt und ist mit einem Ende an einem Spannhebel 30 angeordnet. Das Befestigungselement 22 ist im gezeigten Ausführungsbeispiel in Form einer Schlaufe vorhanden. Der Spann-hebel 30 befindet sich in der ersten Position, in der der passive Aktuator 14 gespannt wird. Wird der Spannhebel 30 entlang des Pfeils 32 bewegt, wird der passive Aktuator 14 entspannt und somit in den zweiten Zustand überführt.

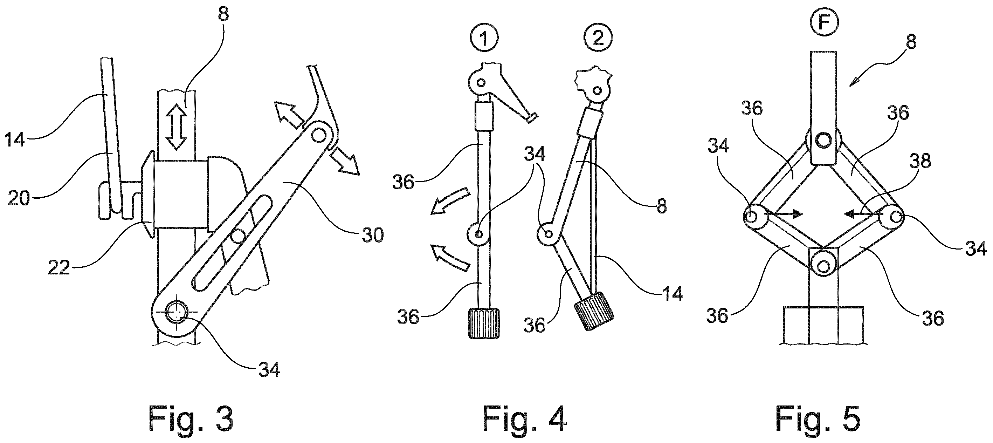

Die Figuren 3, 4 und 5 zeigen weitere Ausführungsbeispiele für entsprechende Betätigungselemente. In Figur 3 ist am Kraftübertragungselement 8 das Befestigungselement 22 verschiebbar gelagert, an dem sich ein Ende 20 des passiven Aktuators 14 befindet. Über einen Spannhebel 30, der um eine Schwenkachse 34 schwenkbar am Kraftübertragungselement 8 angeordnet ist, kann das Befestigungselement 22 verschoben werden und so der passive Aktuator 14 in den ersten Zustand oder den zweiten Zustand gebracht werden.

In Figur 4 ist eine weitere Ausführungsform gezeigt. Das Kraftübertragungselement 8 besteht aus Teilelementen 36, die um eine Schwenkachse 34 herum schwenkbar aneinander gelagert sind. Im linken Teil der Figur 4 bilden die beiden Teilelemente 36 eine gerade Ausführungsform des Kraftübertragungselementes 8, wodurch ein parallel zum Kraftübertragungselement angeordneter passiver Aktuator 14 gestreckt und damit gespannt wird. Im rechten Teil der Figur 4 ist das Kraftübertragungselement 8 eingeknickt, so dass sich die Länge des passiven Aktuators 14 verkürzt und der Aktuator so entspannt wird. Im linken Teil der Figur 4 befindet sich der Aktuator 14 folglich im ersten Zustand und im rechten Teil der Figur 4 im zweiten Zustand.

Figur 5 zeigt eine weitere Ausführungsform, bei der im Kraftübertragungselement 8 vier Teilelemente 36 angeordnet sind, die um zwei Gelenkachsen 34 schwenkbar gelagert sind. Dazwischen befindet sich ein Spannelement 38, durch das der Ab-stand zwischen den beiden Schwenkachsen 34 verändert werden kann. Im gezeigten Ausführungsbeispiel befindet sich ein parallel zum Kraftübertragungselement 8 angeordneter passiver Aktuator im zweiten Zustand. Wird der Abstand zwischen den beiden Schwenkachsen 34 verringert, wird die Ausdehnung in der Figur 5 von oben nach unten des Kraftübertragungselementes 8 vergrößert und der parallel angeordnete passive Aktuator 14 gespannt und somit in den ersten Zustand überführt.

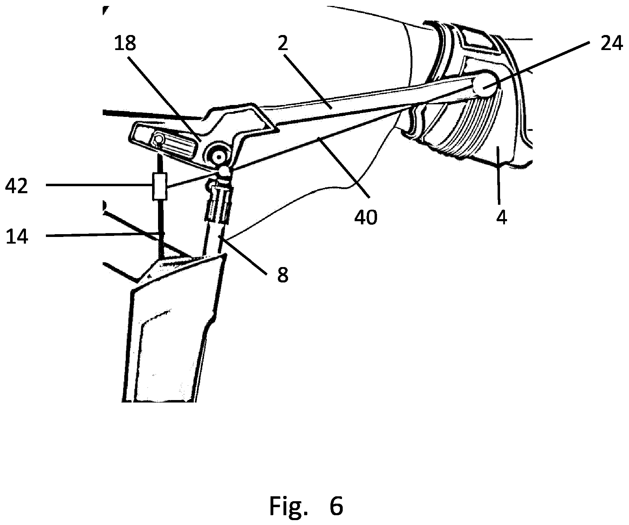

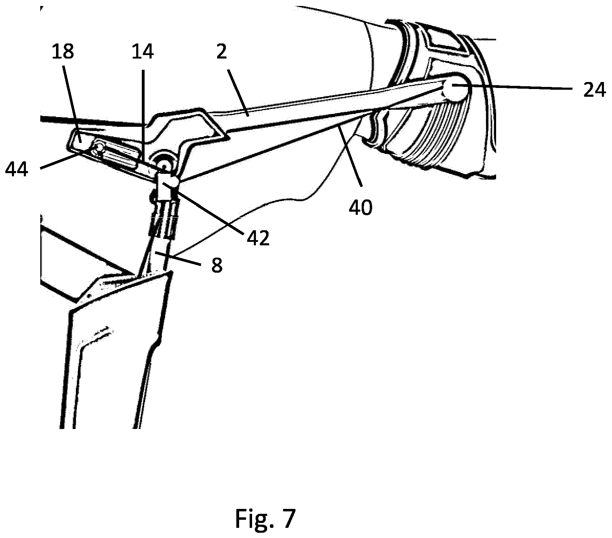

Figur 6 zeigt eine weitere alternative Ausgestaltung. Der passive Aktuator 14 greift am Hebelelement 18, das mit dem Armstützelement 2 verbunden ist, an. Das Armstützelement 2 ist am Kraftübertragungselement 8 schwenkbar angeordnet. Das Betätigungselement 24 befindet sich im gezeigten Ausführungsbeispiel an der Armschale 4 und ist eingerichtet, ein Zugelement 40 aufzuwickeln. Über einen Verbinder 42 ist das Zugelement 40 am passiven Aktuator 14 angeordnet. In Figur 6 befindet sich der Aktuator 14 im ersten Zustand. Die Zugkraft, die vom passiven Aktuator 14 auf das Hebelelement 18 ausgeübt wird, führt zu einem Drehmoment um die Schwenkachse, um die das Armstützelement 2 am Kraftübertragungselement 8 angeordnet ist. Dadurch wird eine unterstützende Kraft auf das Armstützelement 2 ausgeübt. Figur 7 zeigt die Darstellung mit dem Aktuator 14 im zweiten Zustand. Das Betätigungselement 24 hat einen Teil des Zugelementes 40 aufgewickelt und so eine Zugkraft auf den Verbinder 42 ausgeübt. Man erkennt, dass der obere Teil des passiven Aktuators 14, der an dem Hebelelement 18 angreift, nun direkt auf die Schwenkachse gerichtet ist, um die das Abstützelement 2 relativ zum Kraftübertragungselement 8 schwenkbar ist. Die ausgeübte Kraft führt folglich nicht zu einem Drehmoment.

Man erkennt zudem in den Figuren 6 und 7, dass ein Angriffspunkt 44 in einem dafür vorgesehenen Langloch verschiebbar ausgebildet ist. Dadurch lässt sich ein Betrag der Unterstützungskraft einstellen.

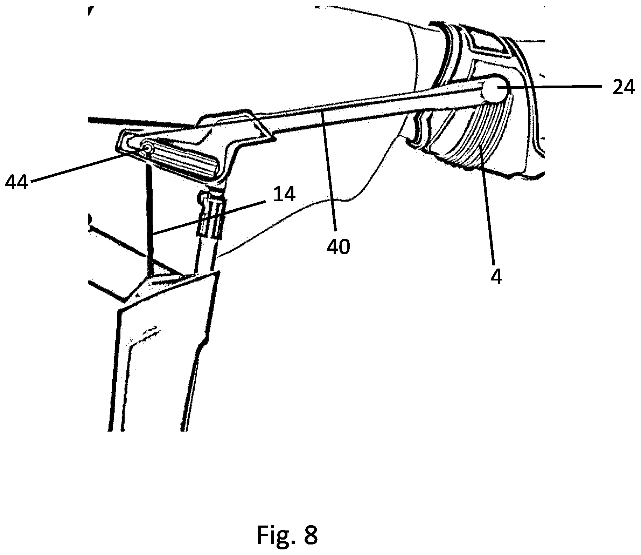

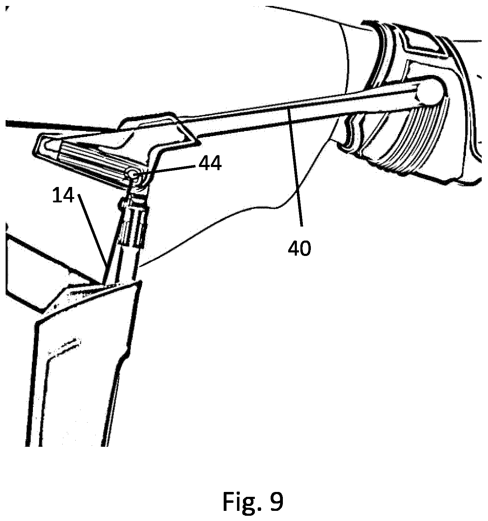

Figur 8 zeigt eine alternative Ausführungsform zur Darstellung in den Figuren 6 und 7. Auch hier ist das Zugelement 40 mit dem Betätigungselement 24 an der Armschale 4 verbunden. Allerdings greift das Zugelement 40 nun am Angriffspunkt 44 an, an dem auch der passive Aktuator 14 angreift. **In** Figur 8 befindet sich der passive Aktuator 14 im ersten Zustand. Wird nun die vom Zugelement 40 ausgeübte Zugkraft reduziert, sorgt eine weitere Feder, die nicht dargestellt ist, dafür, dass der Angriffspunkt 44 auf die Schwenkachse hin verschoben wird. Dadurch wird die aufgebrachte Kraft reduziert und der passive Aktuator 14 befindet sich im zweiten Zustand. Dies ist in Figur 9 dargestellt.

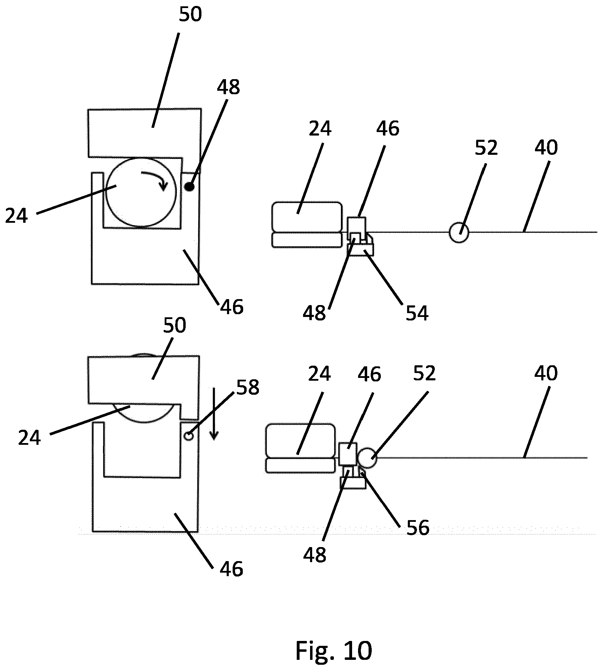

Figur 10 zeigt schematische Ansichten einer Sicherungseinrichtung wie sie bei einer Vorrichtung der hier beschriebenen Art verwendet werden kann.

Im jeweils linken Teil der Figur 10 befindet sich eine schematische Draufsicht auf das Betätigungselement 24, das beispielsweise als Drehknopf oder sogenanntes "BOA"-Element ausgebildet sein kann. Im jeweils linken Teil der Figur 10 ist das Betätigungselement 24 in einer Seitenansicht dargestellt. Das Betätigungselement 24 ist eingerichtet, dass im jeweils rechten Teil der Figur 10 dargestellte Zugelement 40 aufzuwickeln oder abzuwickeln und so den Aktuator vom ersten Zustand in den zweiten Zustand zu bringen. Der obere Teil von Figur 10 zeigt schematisch die Situation bei angelegter Vorrichtung und geschlossener Armschale 4, die in Figur 10 aus Übersichtlichkeitsgründen nicht dargestellt ist. Die Armschale 4 wird im gezeigten Ausführungsbeispiel über einen Gurt geschlossen, dessen Stecker 46 schematisch dargestellt ist. Im oberen Teil der Figur 10, oben links, ist der Stecker 46 im geschlossenen Zustand dargestellt. Er ist über einen Pin 48, der in der linken oberen Darstellung von Figur 10 senkrecht zur Zeichenebene verläuft, gesichert. In diesem Zustand drückt der Stecker 46 eine Schutzkappe 50, die ebenfalls an der Armschale angeordnet sein kann, in die oben links dargestellte Position, sodass das Betätigungselement 24 zugänglich ist. In dieser Situation kann das Betätigungselement 24 betätigt werden und das Zugelement 40 bewegt werden. Am Zugelement 40 befindet sich eine Kugel 52 die mitbewegt wird, sobald das Zugelement 40 durch das Betätigungselement 24 aufgewickelt oder abgewickelt wird. Der Pin 48 ist im rechten Teil der Figur 10 ebenfalls dargestellt. Er befindet sich an einem Trägerelement 54. Man erkennt im oberen Teil der Figur 10, dass der Pin 48 in dem schematisch dargestellten Teil des Steckers 46 eingreift und so verhindert, dass der Stecker 46 bewegt werden kann.

Im unteren Teil der Figur 10 ist eine andere Situation dargestellt. Im rechten Teil der unteren Hälfte der Figur 10 ist gezeigt, dass die Kugel 52 nach rechts bewegt wurde, indem das Betätigungselement 24 betätigt und das Zugelement 40 aufgewickelt wurde. Dabei kommt die Kugel 52 mit einem Sicherungselement 56 in Kontakt, das eine schräge Kontaktfläche aufweist. Die Kugel 52 wird durch betätigen des Betätigungselementes 24 nach links bewegt und drückt aufgrund der schrägen Kontaktfläche das Sicherungselement 56 und damit auch das Trägerelement 54 und den sich darin befindlichen Pin 48 nach unten. Dadurch kommt er außer Eingriff mit dem Stecker 46. Dies ist im linken unteren Teil der Figur 10 durch die leer dargestellte Eingriffsöffnung 58 dargestellt. In diesem Zustand kann der Stecker 46 wie durch den Feil im unteren Teil von Figur 10 dargestellt ist, nach unten bewegt werden und so die nicht dargestellte Armschale 4 geöffnet werden. Dadurch verschiebt sich die Schutzkappe 50 in den unteren Teil von Figur 10 dargestellte Positionen, sodass das Betätigungselement 24 nicht mehr zugänglich ist und nicht bewegt werden kann.

Durch diese Sicherungseinrichtung werden unterschiedliche Effekte erreicht.

Das Betätigungselement 24 kann nur betätigt werden, wenn sich die Schutzkappe 50 in der im oberen Teil der Figur 10 gezeigten Position befindet. Dies ist jedoch nur dann möglich, wenn sie durch den Stecker 46 verschoben wurde und dieser bevorzugt durch den Pin 48 arretiert wird. Der Aktuator 14 ist folglich nur dann durch das Betätigungselement 24 in den ersten Zustand zu bringen, wenn der Stecker 46 geschlossen ist und die Armschale 4 somit sicher am Arm des Trägers anliegt. Anderenfalls ist es in der gezeigten bevorzugten Ausgestaltung nicht möglich, den passiven Aktuator in den ersten Zustand und damit die ausgeübte Kraft auf ihr Maximallevel anzuheben.

Durch das Zusammenwirken von Kugel 52 und Sicherungselement 56 im gezeigten Ausführungsbeispiel wird zudem erreicht, dass der Stecker 46 nur dann entfernt und damit die Armschale 4 geöffnet werden kann, wenn sich die Kugel 52 in der im unteren Teil der Figur 10 gezeigten Position befindet, also eine Endposition erreicht hat. Diese ist vorteilhafterweise der zweite Zustand, in dem die von dem passiven Aktuator auf das Armstützelement ausgeübte Kraft minimal ist.

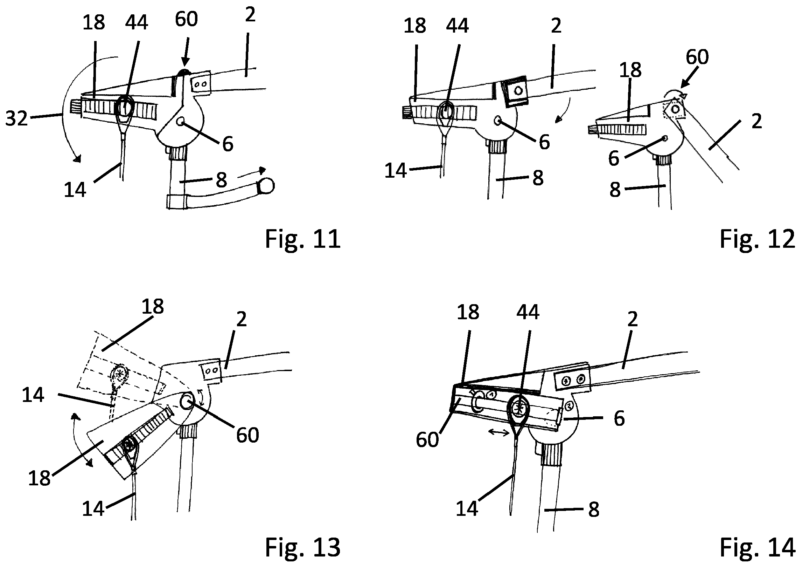

Figur 11 zeigt einen Ausschnitt aus der Vorrichtung gemäß einem weiteren Ausführungsbeispiel der vorliegenden Erfindung. Das Armstützelement 2 ist an dem Kraftübertragungselement 8 durch das Gelenk 6 schwenkbar angeordnet. Mit dem Armstützelement 2 ist das Hebelelement 18 verbunden, an dem an dem Angriffspunkt 44 der passive Aktuator 14 angreift. Durch ein Koppelgelenk 60 ist das Hebelelement 18 von dem Armstützelement 2 lösbar oder zumindest in Richtung des Pfeils 32 abklappbar. Dadurch wird der passive Aktuator 14 entspannt und so keine Unterstützungskraft mehr auf das Armstützelement 2 übertragen. Beim erneuten Koppeln des Hebelelementes 8 mit dem Armstützelement 2 kann das Gelenk 6 mittels des an dem Kraftübertragungselement 8 angeordneten Zugband festgehalten wer-den.

Figur 12 zeigt einen Ausschnitt aus der Vorrichtung gemäß einem weiteren Ausführungsbeispiel der vorliegenden Erfindung. Dabei befindet sich die Vorrichtung im linken Teil der Figur 12 im ersten Zustand und im rechten Teil der Figur 12 im zweiten Zustand. Man erkennt in beiden Teilen das Kraftübertragungselement 8, das Armstützelement 2 sowie das dazwischen liegende Schwenkgelenk 6 mit der Schwenkachse, um die die beiden Elemente um einander verschwenkbar sind. Zudem ist das Hebelelement 18 dargestellt, an dem der passive Aktuator 14 an einem Angriffspunkt 44 angreift. Zwischen dem Hebelelement 18 und dem Armstützelement 2 befindet sich ein Koppelgelenk 60, das verriegelbar ist. Im linken Teil der Figur 12 ist das Koppelgelenk 60 verriegelt, so dass das Armstützelement 2 nicht relativ zum Hebelelement 18 verschwenkt werden kann. Die vom Aktuator 14 auf das Hebelelement 18 übertragene Kraft, die zum gewünschten Drehmoment um die Schwenkachse längs 6 führt, wird folglich durch das verriegelte Koppelgelenk 60 auf das Armstützelement 2 übertragen. Im rechten Teil der Figur 12 ist das Koppelgelenk entriegelt, so dass das Armstützelement 2 relativ zum Hebelgelenk 18 verschwenkbar ist. Die von dem aus Übersichtlichkeitsgründen im rechten Teil der Figur 12 nicht gezeigten passiven Aktuator aufgebrachte Kraft wird folglich nicht auf das Armstützelement 2 übertragen, so dass in diesem Fall der Aktuator in den zweiten Zustand gebracht wurde.

Figur 13 zeigt eine ähnliche Konstruktion, bei der das Hebelelement 18 aus einer ersten Position, die mit gestrichelten Linien dargestellt ist und in der sich der Aktuator 14 im ersten Zustand befindet, in eine Zweitposition bringbar ist, in der das Hebelelement 18 mit durchgezogenen Linien dargestellt ist. In diesem Fall befindet sich der passive Aktuator 14 im zweiten Zustand, in dem er gegenüber dem ersten Zustand entspannt ist, so dass eine deutlich geringere oder gar keine Kraft auf das Hebelelement 18 übertragen wird. Das Koppelgelenk 60 befindet sich im gezeigten Ausführungsbeispiel in Überdeckung mit dem Schwenkgelenk 6. Auch hier wird eine verriegelbare und entriegelbare Verbindung zwischen dem Hebelelement 18 und dem Armstützelement 2 hergestellt.

Figur 14 zeigt eine andere Ausgestaltung. Auch hier ist das Hebelelement 18 mit dem Armstützelement 2 verbunden, und gemeinsam mit diesem um eine Schwenk-achse des Schwenkgelenkes 6 relativ zum Kraftübertragungselement 8 verschwenkbar. Das Hebelelement 18 ist jedoch nicht relativ zu dem Armstützelement 2 bewegbar. Der Angriffspunkt 44, an dem der passive Aktuator 14 am Hebelelement 18 angreift, ist entlang einer Schiene 62 verschiebbar ausgebildet. Dadurch kann der Abstand zwischen dem Angriffspunkt 44 und der Schwenk-achse des Schwenkgelenkes 6 variiert werden, wodurch sich der Hebelarm der von dem Aktuator 14 auf das Hebelelement 18 aufgebrachten Kraft relativ zu dieser Schwenk-achse verkürzen oder verlängern lässt. Dadurch wird auch das aufgebrachte Drehmoment und somit schließlich auch die vom Aktuator 14 auf das Armstützelement 2 aufgebrachte Unterstützungskraft variiert. Wird im gezeigten Ausführungsbeispiel der Figur 14 der Angriffspunkt 44 bis an den rechten Anschlag verschoben, befindet er sich direkt auf der Schwenkachse des Schwenkgelenkes, so dass kein Drehmoment mehr übertragen wird.

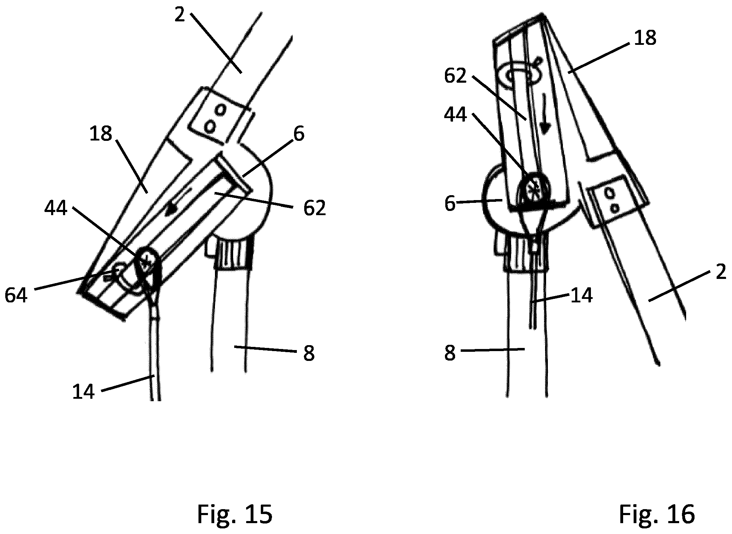

Die Figuren 15 und 16 zeigen eine Ausgestaltung der Vorrichtung aus Figur 14, bei der das Betätigungselement so ausgebildet ist, dass es automatisch von der Vorrichtung betätigt wird und in Abhängigkeit der Position des Armstützelementes 2 relativ zum Kraftübertragungselement 8 den Aktuator in den ersten Zustand oder den zweiten Zustand bringt.

Die Konstruktion entspricht im Wesentlichen der in Figur 14 gezeigten Ausführungsform, mit dem Unterschied, dass der Angriffspunkt 44 nicht arretierbar ist, sondern frei auf der Schiene 62 entlanggleiten kann. In Figur 15 ist die Situation eines angehobenen Armes dargestellt. Man erkennt, dass das Armstützelement 2 vom Hebelelement 18 aus nach oben gerichtet ist. Durch die vom Aktuator 14 aufgebrachte Kraft gleitet der Angriffspunkt 44 in die gezeigte Position, bis er an einem Anschlagsring 64 anschlägt. Selbstverständlich sind auch andere Arten eines Anschlages möglich. Wichtig ist lediglich, dass die Bewegung des Angriffspunktes 44 entlang der Schiene 62 in diese Richtung durch einen Anschlag begrenzt wird. Der Anschlag ist beispielsweise in der Form des gezeigten Anschlagsringes 64 entlang der Schiene 62 verschiebbar und an dieser oder auf dieser Schiene 62 arretierbar. Durch die vom Aktuator aufgebrachte Kraft bewegt sich folglich der Angriffspunkt 44 auf die maximal von der Schwenkachse des Schwenkgelenkes 6 entfernte Position. Das aufgebrachte Drehmoment und damit auch die auf das Armstützelement 2 aufgebrachte Kraft wird auf diese Weise maximiert. Vorzugsweise ist der Angriffspunkt in wenigstens einer gewünschten Position, vorzugsweise in jeder gewünschten Position arretierbar, so dass er ohne die Arretierung zu lösen nicht an der Schiene 62 entlanggleiten kann. Die Arretierung kann beispielsweise durch Klemmbacken, eine Scherenklemme, magnetisch oder durch ein Arretierungselement, beispiels-weise einen in eine Arretierungsbohrung einschiebbaren Stift erfolgen.

Figur 16 zeigt die Situation bei abgesenktem Arm. Man erkennt, dass das Armstützelement 2 vom Schwenkgelenk 6 aus nach unten zeigt. In diesem Fall wird der Angriffspunkt 44 durch die vom Aktuator 14 aufgebrachte Kraft in die entgegengesetzte Richtung verschoben, wie dies in Figur 16 dargestellt ist. Auch hier befindet sich ein Anschlag, der das Verschieben und die Bewegung des Angriffspunktes 44 begrenzt. Im gezeigten Ausführungsbeispiel ist der Angriffspunkt 44 in dieser Situation nicht nur näher an der Schwenkachse des Schwenkgelenkes, was prinzipiell ausreichen würde, um einen zweiten Zustand zu definieren, sondern er befindet sich in Überdeckung mit der Schwenkachse, so dass das ausgebrachte Drehmoment verschwindet.

### Bezugszeichenliste

- 2: Armstützelement
- 4: Armschale
- 6: Schwenkgelenk
- 8: Kraftübertragungselement
- 10: unteres Ende
- 12: Gegenlagerelement
- 14: passiver Aktuator
- 16: Bowdenzug
- 18: Hebelelement
- 20: Ende
- 22: Befestigungselement
- 24: Betätigungselement
- 26: Befestigungsposition
- 28: Umlenkrolle
- 30: Spannhebel
- 32: Pfeil
- 34: Schwenkachse
- 36: Teilelement
- 38: Spannelement
- 40: Zugelement
- 42: Verbinder
- 44: Angriffspunkt
- 46: Stecker
- 48: Pin
- 50: Schutzkappe
- 52: Kugel
- 54: Trägerelement
- 56: Sicherungselement
- 58: Eingriffsöffnung
- 60: Koppelgelenk
- 62: Schiene
- 64: Anschlagsring

## Patentansprüche

1. Vorrichtung zum Unterstützen wenigstens eines Armes eines Benutzers, wobei die Vorrichtung
a. wenigstens ein Armstützelement (2) mit je einer Armschale (4) zum Anlegen an jeweils einen Arm,
b. wenigstens einen passiven Aktuator (14),
i. der als elastisches Element ausgebildet ist und
ii. der eingerichtet ist, eine Kraft auf das wenigstens eine Armstützelement (2) auszuüben, durch die im angelegten Zustand der Vor-richtung eine Aufwärtsbewegung des Arms in der Armschale (4) unterstützt wird, und
c. wenigstens ein Gegenlager für die aufzubringende Kraft aufweist, wobei die Vorrichtung wenigstens ein Betätigungselement (24) aufweist, durch dessen Betätigen der Aktuator (14) in einen ersten Zustand, in dem der Aktuator (14) die Kraft auf das wenigstens eine Armstützelement (2) ausübt, und in einen zweiten Zustand bringbar ist, in dem er eine kleinere oder keine Kraft auf das Armstützelement (2) ausübt, **dadurch gekennzeichnet, dass** durch das Betätigen des Betätigungselementes (24) eine Richtung der vom Aktuator (14) aufgebrachten Kraft veränderbar ist, ohne dass ein Ende des elastischen Elementes verschoben wird.

2. Vorrichtung nach Anspruch 1, **dadurch gekennzeichnet, dass** der Aktuator (14) im ersten Zustand eine maximale Kraft auf das wenigstens eine Armstützelement (14) ausübt.

3. Vorrichtung nach Anspruch 1 oder 2, **dadurch gekennzeichnet, dass** der Aktuator (14) durch Betätigen des wenigstens einen Betätigungselementes (24) in mehreren Stufen oder stufenlos von dem ersten Zustand in den zweiten Zustand bringbar ist, wobei die auf das wenigstens eine Armstützelement (2) durch den Aktuator (14) ausgeübte Kraft abnimmt.

4. Vorrichtung nach Anspruch 3, **dadurch gekennzeichnet, dass** der Aktuator (14) durch Betätigen des wenigstens einen Betätigungselementes (24) in wenigstens einen Zwischenzustand, bevorzugt mehrere Zwischenzustände, bringbar ist, sodass die auf das wenigstens eine Armstützelement (2) durch den Aktuator (14) ausgeübte Kraft in Stufen oder stufenlos einstellbar ist.

5. Vorrichtung nach einem der vorstehenden Ansprüche, **dadurch gekennzeichnet, dass** der wenigstens eine Aktuator (14) wenigstens ein elastisches Element aufweist, das entspannbar ist, indem der Aktuator (14) durch Betätigen des wenigstens einen Betätigungselement (24) aus dem ersten Zustand in den zweiten Zustand gebracht wird.

6. Vorrichtung nach Anspruch 5, **dadurch gekennzeichnet, dass** das elastische Element ein Ende (20) mit einem Befestigungselement (22) aufweist, das durch Betätigen des wenigstens einen Betätigungselementes (24) bewegbar, insbesondere verschiebbar oder verschwenkbar ist.

7. Vorrichtung nach einem der vorstehenden Ansprüche, **dadurch gekennzeichnet, dass** das Gegenlager wenigstens ein Gegenlagerelement (12) und wenigstens ein Kraftübertragungselemente (8) aufweist, wobei das wenigstens eine Armstützelement (2) um eine Schwenkachse schwenkbar an dem wenigstens einen Kraftübertragungselement (8) angeordnet ist und wobei eine Kraftrichtung, in die die von dem Aktuator (14) ausgeübte Kraft wirkt, im zweiten Zustand des Aktuators näher an der Schwenkachse verläuft als im ersten Zustand des Aktuators.

8. Vorrichtung nach Anspruch 7, **dadurch gekennzeichnet, dass** die Kraftrichtung im zweiten Zustand durch die Schwenkachse verläuft.

9. Vorrichtung nach Anspruch 7 oder 8, **dadurch gekennzeichnet, dass** der Aktuator (14) ein elastisches Element aufweist, an dem wenigstens ein Zugelement (40) angeordnet ist, auf das durch Betätigen des Betätigungselementes (24) eine Zugkraft ausübbar ist, wenn sich der Aktuator (14) im ersten Zustand befindet.

10. Vorrichtung nach einem der vorstehenden Ansprüche, **dadurch gekennzeichnet, dass** die Armschalen (4) ein Verschlusselement aufweisen, durch das die Armschalen (4) um den Arm des Benutzers schließbar sind.

11. Vorrichtung nach Anspruch 10, **dadurch gekennzeichnet, dass** das Verschlusselement nur geöffnet werden kann, wenn sich der Aktuator (14) im zweiten Zustand befindet.

12. Vorrichtung nach einem der vorstehenden Ansprüche, **dadurch gekennzeichnet, dass** die Vorrichtung zwei Armstützelemente (2) mit je einer Armschale (4) zum Anlegen an jeweils einen Arm und vorzugsweise zwei Kraftübertragungselemente (8) aufweist.

## Claims

1. A device for supporting at least one arm of a user, wherein the device has
a. at least one arm support element (2), each of which has an arm shell (4) for mounting on an arm,
b. at least one passive actuator (14),
i. which is designed as an elastic element and
ii. which is configured to exert a force on the at least one arm support element (2), by way of which an upward movement of the arm in the arm shell (4) is supported when the device is in the mounted state, and
c. at least one counter bearing for the force to be applied,
wherein the device comprises at least one actuating element (24), the actuation of which allows the actuator (14) to be moved into a first state, in which the actuator (14) exerts the force on the at least one arm support element (2), and into a second state, in which it exerts a smaller or no force on the arm support element (2)
**characterized in that**
by actuating the actuating element the direction of the force, exerted by the actuator (14) is changeable without moving an end of the elastic element.

2. The device according to claim 1, **characterized in that** the actuator (14) exerts a maximum force on the at least one arm support element (14) in the first state.

3. The device according to claim 1 or 2, **characterized in that** the actuator (14) can be moved from the first state into the second state in several steps or continuously by actuating the at least one actuating element (24), wherein the force exerted by the actuator (14) on the at least one arm support element (2) decreases.

4. The device according to claim 3, **characterized in that** the actuator (14) can be brought into at least one intermediate state, but preferably several intermediate states, by actuating the at least one actuating element (24), so that the force exerted by the actuator (14) on the at least one arm support element (2) can be adjusted in steps or continuously.

5. The device according to one of the preceding claims, **characterized in that** the at least one actuator (14) features at least one elastic element that can be relaxed by moving the actuator (14) out of the first state into the second state by actuating the at least one actuating element (24).

6. The device according to claim 5, **characterized in that** the elastic element has an end (20) with a fastening element (22) that can be moved, especially swivelled or displaced, by actuating the at least one actuating element (24).

7. The device according to one of the preceding claims, **characterized in that** the counter bearing features at least one counter bearing element (12) and at least one force transmission element (8), wherein the at least one arm support element (2) is arranged on the at least one force transmission element (8) such that it can be swivelled about a swivel axis, and wherein a force direction, in which the force exerted by the actuator (14) acts, runs closer to the swivel axis in the second state of the actuator than in the first state.

8. The device according to claim 7, **characterized in that** the force direction passes through the swivel axis in the second state.

9. The device according to claim 7 or 8, **characterized in that** the actuator (14) features an elastic element, on which at least one tension element (40) is arranged, to which a tensile force can be exerted by actuating the actuating element (24) when the actuator (14) is in the first state.

10. The device according to one of the preceding claims, **characterized in that** the arm shells (4) comprise a closing element, by way of which the arm shells (4) can be closed around the user's arm.

11. The device according to claim 10, **characterized in that** the fastening element can only be opened when the actuator (14) is in the second state.

12. The device according to one of the preceding claims, **characterized in that** the device comprises two arm support elements (2), each of which has an arm shell (4) for mounting on an arm, and preferably two force transmission elements (8).

## Revendications

1. Dispositif destiné à soutenir au moins un bras d'un utilisateur, le dispositif comprenant
a. au moins un élément de soutien de bras (2) ayant chacun une coque pour bras (4) destinée à être appliquée contre un bras respectif,
b. au moins un actionneur passif (14),
i. qui est réalisé sous la forme d'un élément élastique et
ii. qui est conçu pour exercer une force sur ledit au moins un élément de soutien de bras (2), grâce à laquelle, à l'état appliqué du dispositif, un mouvement vers le haut du bras dans la coque pour bras (4) est assisté, et
c. au moins un contre-appui pour la force à appliquer,
dans lequel le dispositif comporte au moins un élément d'actionnement (24) dont l'actionnement permet d'amener l'actionneur (14) dans un premier état dans lequel l'actionneur (14) exerce la force sur ledit au moins un élément de soutien de bras (2), et dans un deuxième état dans lequel il exerce une force moindre, voire aucune force, sur l'élément de soutien de bras (2),
**caractérisé en ce que**
l'actionnement de l'élément d'actionnement (24) permet de modifier la direction de la force exercée par l'actionneur (14), sans déplacer une extrémité de l'élément élastique.

2. Dispositif selon la revendication 1,
**caractérisé en ce que** l'actionneur (14) exerce, dans le premier état, une force maximale sur ledit au moins un élément de soutien de bras (2).

3. Dispositif selon la revendication 1 ou 2,
**caractérisé en ce que** l'actionneur (14) peut être amené, par l'actionnement dudit au moins un élément d'actionnement (24), par étapes ou en continu, du premier état au deuxième état, la force exercée par l'actionneur (14) sur ledit au moins un élément de soutien de bras (2) diminuant alors.

4. Dispositif selon la revendication 3,
**caractérisé en ce que** l'actionneur (14) peut être amené, par l'actionnement dudit au moins un élément d'actionnement (24), dans au moins un état intermédiaire, de préférence dans plusieurs états intermédiaires, de sorte que la force exercée par l'actionneur (14) sur ledit au moins un élément de soutien de bras (2) soit réglable par étapes ou en continu.

5. Dispositif selon l'une des revendications précédentes,
**caractérisé en ce que** ledit au moins un actionneur (14) comporte au moins un élément élastique qui peut être détendu du fait que l'actionneur (14) est amené du premier état au deuxième état par l'actionnement dudit au moins un élément d'actionnement (24).

6. Dispositif selon la revendication 5,
**caractérisé en ce que** l'élément élastique comporte une extrémité (20) munie d'un élément de fixation (22) qui peut être déplacé, en particulier en coulissement ou en pivotement, par l'actionnement dudit au moins un élément d'actionnement (24).

7. Dispositif selon l'une des revendications précédentes,
**caractérisé en ce que** le contre-appui comporte au moins un élément de contre-appui (12) et au moins un élément de transmission de force (8), ledit au moins un élément de soutien de bras (2) étant monté sur ledit au moins un élément de transmission de force (8) de manière à pouvoir pivoter autour d'un axe de pivotement, et une direction de force, dans laquelle agit la force exercée par l'actionneur (14), s'étend plus près de l'axe de pivotement dans le deuxième état de l'actionneur que dans le premier état de l'actionneur.

8. Dispositif selon la revendication 7,
**caractérisé en ce que**, dans le deuxième état, la direction de la force passe par l'axe de pivotement.

9. Dispositif selon la revendication 7 ou 8,
**caractérisé en ce que** l'actionneur (14) comporte un élément élastique sur lequel est disposé au moins un élément de traction (40) sur lequel une force de traction peut être exercée par l'actionnement de l'élément d'actionnement (24), lorsque l'actionneur (14) se trouve dans le premier état.

10. Dispositif selon l'une des revendications précédentes,
**caractérisé en ce que** les coques pour bras (4) comportent un élément de fermeture permettant de fermer les coques pour bras (4) autour du bras de l'utilisateur.

11. Dispositif selon la revendication 10,
**caractérisé en ce que** l'élément de fermeture ne peut être ouvert que lorsque l'actionneur (14) se trouve dans le deuxième état.

12. Dispositif selon l'une des revendications précédentes,
**caractérisé en ce que** le dispositif comporte deux éléments de soutien de bras (2), munis chacun d'une coque pour bras (4) destinée à être appliquée contre un bras respectif, et de préférence deux éléments de transmission de force (8).
